# EUROPEAN PATENT APPLICATION

(11) **EP 3 339 942 A1**
(43) Date of publication of application: **27.06.2018**
(21) Application number: 17209465.8
(22) Date of filing: 21.12.2017
(51) Int. Cl.: G02C 7/04, G02C 7/08, A61B 3/113

(54) **CAPACITIVE SENSING CIRCUITS AND METHODS FOR DETERMINING EYELID POSITION USING THE SAME**

(30) Priority: 21.12.2016 US 201615386021
(71) Applicant: Johnson & Johnson Vision Care, Inc., Jacksonville, FL 32256 (US)
(72) Inventor: BARROWS, Corey Kenneth, Williston, VT Vermont 05495 (US); BUSH, John Michael, Queen Creek, AZ Arizona 85142 (US); HOGGARTH, Steven Philip, Morrisville, NC North Carolina 27560 (US); TONER, Adam, Jacksonville, FL Florida 32256 (US)
(74) Representative: Carpmaels & Ransford LLP

(57) **Abstract**

The present disclosure relates to sensor systems for electronic ophthalmic devices. In certain embodiments, the sensor systems may comprise a first electrode configured to be selectively overlaid by one or more of an upper eyelid and a lower eyelid of a user, a second electrode configured to be selectively overlaid by one or more of an upper eyelid and a lower eyelid of a user, and a system controller cooperatively associated with the first electrode and the second electrode to receive a capacitance measurement therefrom, the system controller configured to determine a position of one or more of the upper eyelid and the lower eyelid in spatial coordinates based on the capacitance measurement received from the first electrode and the second electrode.

## Description

### BACKGROUND OF THE DISCLOSURE

### 1. Field of the Disclosure

The present disclosure relates to electronic ophthalmic devices, such as wearable lenses, including contact lenses, implantable lenses, including intraocular lenses (IOLs) and any other type of device comprising optical components, and more particularly, to sensors and associated hardware and software for determining eyelid position in an individual to activate and control electronic ophthalmic devices.

### 2. Discussion of the Related Art

Lenses, such as contact lenses and intraocular lenses, currently are utilized to correct vision defects such as myopia (nearsightedness), hyperopia (farsightedness), presbyopia and astigmatism. However, properly designed lenses incorporating additional components may be utilized to enhance vision as well as to correct vision defects.

Conventional contact lenses are polymeric structures with specific shapes to correct various vision problems as briefly set forth above. To achieve enhanced functionality, various circuits and components have to be integrated into these polymeric structures. For example, control circuits, microprocessors, communication devices, power supplies, sensors, actuators, light-emitting diodes, and miniature antennas may be integrated into contact lenses via custom-built optoelectronic components to not only correct vision, but to enhance vision as well as provide additional functionality as is explained herein.

Electronic and/or powered contract lenses may be designed to provide enhanced vision via zoom-in and zoom-out capabilities, or simply modify the refractive capabilities of the lenses. Electronic and/or powered contact lenses may be designed to enhance color and resolution, to display textural information, to translate speech into captions in real time, to offer visual cues from a navigation system, and to provide image processing and internet access. The lenses may be designed to allow the wearer to see in low-light conditions. The properly designed electronics and/or arrangement of electronics on lenses may allow for projecting an image onto the retina, for example, without a variable-focus optic lens, provide novelty image displays and even provide wakeup alerts.

Additionally or alternately, eyelid position, eye gaze, and/or pupil convergence may be utilized to control the functionality of a contact lens in certain circumstances. When an individual focuses on a near object, for example when reading, his/her pupils converge to fix the gaze of both eyes on the same location. This phenomena is based on the geometry of the system, a triangle being formed by the two eyes and the area of focus, and attention being brought to a specific, nearby object. This effect is used in the design of spectacles, stereoscopes, and related instruments to ensure clear and comfortable vision when gazing at nearby objects. This effect may also be monitored in a clinical setting, for example by recording a user's pupil positions by observing them with a camera and performing pattern recognition functions. Pupil convergence could also be detected by a similar camera and detection system implemented in spectacle lenses. However, such clinical methods may not be suitable for non-clinical settings, such as a everyday wear.

Accordingly, there exists a need for a means and method for detecting certain physiological functions, such as eyelid position, pupil convergence, and gaze direction, and utilizing them to activate and/or control an electronic or powered ophthalmic lens.

### SUMMARY OF THE DISCLOSURE

The present disclosure relates to powered ophthalmic devices that comprise an electronic system and performs a number of functions, including actuating a variable-focus optic if included. The electronic system includes one or more batteries or other power sources, power management circuitry, one or more sensors, sensor configurations, control algorithms, circuitry comprising a capacitive sensor, and lens driver circuitry.

In accordance with another aspect, the present disclosure is directed to powered ophthalmic lenses. Such lenses comprise a contact lens and an eye gaze tracking system incorporated into the contact lens, the eye gaze tracking system including a sensor to determine and track eye position, a system controller cooperatively associated with the sensor, the system controller configured to determine and track gaze direction in spatial coordinates based on information from the sensor output a control signal, and at least one actuator configured to receive the output control signal and implement a predetermined function.

In accordance with yet another aspect, the present disclosure is directed to an eyelid position sensing system for a powered ophthalmic device. The eyelid position sensing system may comprise a first electrode configured to be selectively overlaid by one or more of an upper eyelid and a lower eyelid of a user. The eyelid position sensing system may comprise a second electrode configured to be selectively overlaid by one or more of an upper eyelid and a lower eyelid of a user. The eyelid position sensing system may comprise a system controller cooperatively associated with the first electrode and the second electrode to receive a capacitance measurement therefrom, the system controller configured to determine a position of one or more of the upper eyelid and the lower eyelid in spatial coordinates based on the capacitance measurement received from the first electrode and the second electrode.

In accordance with yet another aspect, the present disclosure is directed to a powered ophthalmic device. The powered ophthalmic device may comprise a lens including an optic zone and a peripheral zone. The powered ophthalmic device may comprise an eye gaze tracking system incorporated into the peripheral zone of the contact lens, the eye gaze tracking system including a capacitive touch sensor to detect a capacitance based at least on a position of one or more of an upper eyelid and a lower eyelid, a system controller cooperatively associated with the capacitive touch sensor, the system controller configured to determine gaze direction in spatial coordinates based on information received from the sensor, and at least one actuator configured to receive the output control signal and implement a predetermined function.

Eye tracking is the process of determining either or both where an individual is looking, point of gaze, or the motion of an eye relative to the head. An individual's gaze direction is determined by the orientation of the head and the orientation of the eyes and/or configuration of eyelid position. More specifically, the orientation of an individual's head determines the overall direction of the gaze while the orientation of the individual's eyes determines the exact gaze direction which in turn is limited by the orientation of the head. Information of where an individual is gazing provides the ability to determine the individual's focus of attention and this information may be utilized in any number of disciplines or application, including cognitive science, psychology, human-computer interaction, marketing research and medical research. For example, eye gaze direction may be utilized as a direct input into a controller or computer to control another action. In other words, simple eye movements may be utilized to control the actions of other devices, including highly complex functions. Simple eye movements may be utilized in a manner similar to "swipes" which have become common in touch-screen and smartphone applications, for example, swiping to unlock a device, change applications, change pages, zoom in or out and the like. Eye gaze tracking systems are presently utilized to restore communication and functionality to those who are paralyzed, for example, using eye movements to operate computers. Eye tracking or gaze tracking may also be utilized in any number of commercial applications, for example, what individuals are paying attention to when they are watching television, browsing websites and the like. The data collected from this tracking may be statistically analyzed to provide evidence of specific visual patterns. Accordingly, information garnered from detecting eye or pupil movement may be utilized in a wide range applications. There are a number of currently available devices for tracking eye movement, including video-based eye trackers, search coils and arrangements for generating electrooculograms. Search coils or inductive sensors are devices which measure the variations of the surrounding magnetic fields. Essentially, a number of coils may be imbedded into a contact lens type device and the polarity and amplitude of the current generated in the coils varies with the direction and angular displacement of the eye. An electrooculogram is generated by a device for the detection of eye movement and eye position based on the difference in electrical potential between electrodes placed on either side of the eye. All of these devices are not suitable for use with a wearable, comfortable electronic ophthalmic lens or powered contact lens. Therefore, in accordance with another exemplary embodiment, the present disclosure is directed to a powered contact lens comprising a gaze sensor incorporated directly into the contact lens.

### BRIEF DESCRIPTION OF THE DRAWINGS

The foregoing and other features and advantages of the disclosure will be apparent from the following, more particular description of preferred embodiments of the disclosure, as illustrated in the accompanying drawings.
Figure 1 illustrates an exemplary ophthalmic device comprising a sensor system in accordance with some embodiments of the present disclosure.
Figure 2 illustrates an exemplary ophthalmic device comprising a sensor system in accordance with some embodiments of the present disclosure.
Figure 3 is a planar view of an ophthalmic device comprising electronic components, including a sensor system and a variable-optic element in accordance with the present disclosure.
Figure 4 is a diagrammatic representation of an exemplary electronic insert, including a combined blink detection and communication system positioned in a powered or electronic ophthalmic device in accordance with some embodiments of the present disclosure.
Figure 5A is a diagrammatic representation of an exemplary electronic system incorporated into a contact lens for detecting eyelid position in accordance with the present disclosure.
Figure 5B is an enlarged view of the exemplary electronic system of Figure 5A
Figure 6 is a diagrammatic representation of an exemplary electronic system incorporated into a contact lens for detecting eyelid position in accordance with the present disclosure.
Figure 7 is a diagrammatic representation of an exemplary electronic system incorporated into a contact lens for detecting eyelid position in accordance with the present disclosure.
Figure 8 is a diagrammatic representation of an exemplary electronic system incorporated into a contact lens for detecting eyelid position in accordance with the present disclosure.
Figure 9A is a diagrammatic representation of an exemplary electronic system incorporated into a contact lens for detecting eyelid position in accordance with the present disclosure.
Figure 9B is a diagrammatic representation of an exemplary electronic system incorporated into a contact lens for detecting eyelid position in accordance with the present disclosure.
Figure 9C is a diagrammatic representation of an exemplary electronic system incorporated into a contact lens for detecting eyelid position in accordance with the present disclosure.
Figure 10A is a diagrammatic representation of an exemplary electronic system incorporated into a contact lens for detecting eyelid position in accordance with the present disclosure.
Figure 10B is a diagrammatic representation of an exemplary electronic system incorporated into a contact lens for detecting eyelid position in accordance with the present disclosure.
Figure 11A is a diagrammatic representation of an exemplary electronic system incorporated into a contact lens for detecting eyelid position in accordance with the present disclosure.
Figure 11B is a diagrammatic representation of an exemplary electronic system incorporated into a contact lens for detecting eyelid position in accordance with the present disclosure.
Figure 11C is a diagrammatic representation of an exemplary electronic system incorporated into a contact lens for detecting eyelid position in accordance with the present disclosure.
Figure 12A is a diagrammatic, front perspective representation of the eyes of an individual gazing at a distant object.
Figure 12B is a diagrammatic, top perspective representation of the eyes of Figure 12A.
Figure 13A is a diagrammatic, front perspective representation of the eyes of an individual gazing at a near object.
Figure 13B is a diagrammatic, top perspective representation of the eyes of 15 Figure 13A.
Figure 14 is a diagrammatic representation of two exemplary pupil position and convergence sensors having a communication channel for synchronizing operation between two eyes in accordance with the present disclosure.
Figure 15A is a diagrammatic representation of an exemplary pupil position and convergence detection system incorporated into a contact lens in accordance with the present disclosure.
Figure 15B is an enlarged view of the exemplary pupil position and convergence detection system of Figure 15A.
Figure 16 is a diagrammatic representation of an exemplary plot of the correlation between pupil convergence and focal distance.
Figure 17A is a diagrammatic, front perspective representation of the eyes of an individual gazing to the right.
Figure 17B is a diagrammatic, top perspective representation of the eyes of Figure 17A.
Figure 18 is a diagrammatic representation of the geometry associated with various gaze directions in two dimensions in accordance with the present disclosure.

### DETAILED DESCRIPTION OF THE PREFERRED EMBODIMENTS

Ophthalmic devices may include contact lenses. Conventional contact lenses are polymeric structures with specific shapes to correct various vision problems as briefly set forth above. To achieve enhanced functionality, various circuits and components may be integrated into these polymeric structures. For example, control circuits, microprocessors, communication devices, power supplies, sensors, actuators, light-emitting diodes, and miniature antennas may be integrated into contact lenses via custom-built optoelectronic components to not only correct vision, but to enhance vision as well as provide additional functionality as is explained herein. Electronic and/or powered contact lenses may be designed to provide enhanced vision via zoom-in and zoom-out capabilities, or simply to modify the refractive capabilities of the lenses. Electronic and/or powered contact lenses may be designed to enhance color and resolution, to display textural information, to translate speech into captions in real time, to offer visual cues from a navigation system, and to provide image processing and internet access. The lenses may be designed to allow the wearer to see in low light conditions. The properly designed electronics and/or arrangement of electronics on lenses may allow for projecting an image onto the retina, for example, without a variable focus optic lens, provide novelty image displays and even provide wakeup alerts. Alternately, or in addition to any of these functions or similar functions, the contact lenses may incorporate components for the noninvasive monitoring of the wearer's biomarkers and health indicators. For example, sensors built into the lenses may allow a diabetic patient to keep tabs on blood sugar levels by analyzing components of the tear film without the need for drawing blood. In addition, an appropriately configured lens may incorporate sensors for monitoring cholesterol, sodium, and potassium levels, as well as other biological markers. This coupled with a wireless data transmitter could allow a physician to have almost immediate access to a patient's blood chemistry without the need for the patient to waste time getting to a laboratory and having blood drawn.

The electronic contact lens of the present disclosure comprises the necessary elements to correct and/or enhance the vision of patients with one or more of the above described vision defects or otherwise perform a useful ophthalmic function. In addition, the electronic contact lens may be utilized simply to enhance normal vision or provide a wide variety of functionality as described above. The electronic contact lens may comprise a variable focus optic lens, an assembled front optic embedded into a contact lens or just simply embedding electronics without a lens for any suitable functionality. The electronic lens of the present disclosure may be incorporated into any number of contact lenses as described above. In addition, intraocular lenses may also incorporate the various components and functionality described herein. However, for ease of explanation, the disclosure will focus on an electronic contact lens to correct vision defects intended for single-use daily disposability.

The present disclosure may be employed in a powered ophthalmic lens or powered contact lens comprising an electronic system, which actuates a variable-focus optic or any other device or devices configured to implement any number of numerous functions that may be performed. The electronic system includes one or more batteries or other power sources, power management circuitry, one or more sensors, clock generation circuitry, control algorithms and circuitry, and lens driver circuitry. The complexity of these components may vary depending on the required or desired functionality of the lens.

A sensor, the components of which may be embedded in a powered contact lens, may detect characteristics of different eye or eyelid position. For example, various signals may include one or more of when an eye is moving up or down, focusing up close, and adjusting to a change in ambient light levels, such as from light to dark, dark to light or any other light condition. As an example, a capacitive sensor may be positioned to detect a position of an eyelid of a user and thereby determine an eye gaze. It is important to note that this list of conditions is exemplary and not exhaustive. In certain embodiments, ophthalmic devices may comprise one or more sensor systems, such as circuits. The sensor systems may be configured with one or more capacitive sensors configured to detect position of an eyelid.

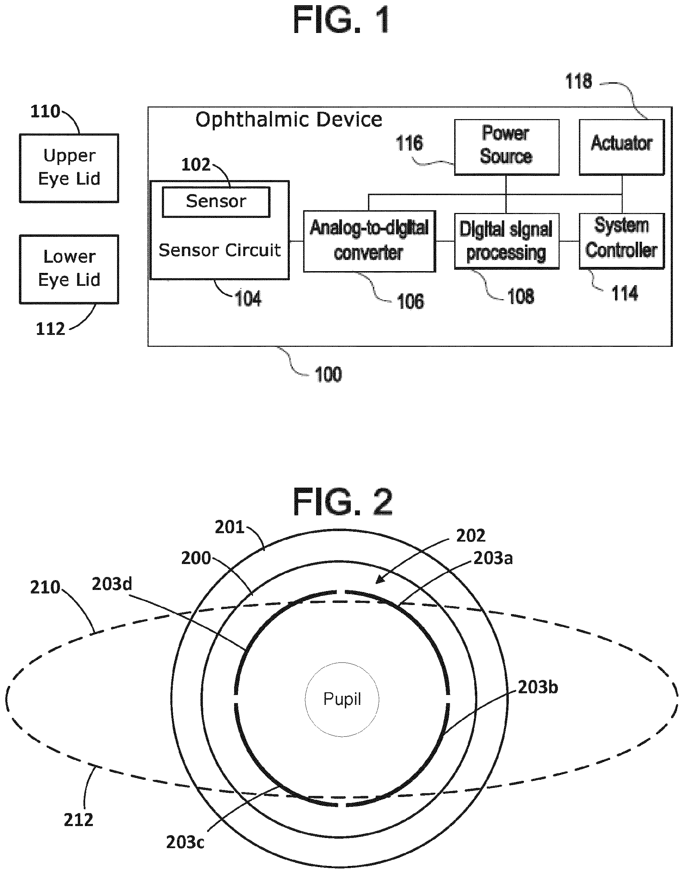

Figure 1 illustrates, in block diagram form, an ophthalmic device 100 disposed on the front surface of the eye or cornea, in accordance with one exemplary embodiment of the present disclosure. Although the ophthalmic device 100 is shown and described as a being disposed on the front surface of the eye, it is understood that other configurations, such as those including intraocular lens configuration may be used. In this exemplary embodiment, the sensor system may comprise one or more of a sensor 102, a sensor circuit 104, an analog-to-digital converter 106, a digital signal processor 108, a power source 116, an actuator 118, and a system controller 114. As illustrated, the ophthalmic device 100 may be disposed adjacent an upper eyelid 110 and a lower eyelid 112, such as adjacent the front eye surface or cornea, wherein the electronic circuitry of the sensor system or circuit 104 may be utilized to implement the sensing of the present disclosure. The sensor 102 as well as the other circuitry is configured to sense various electrical characteristics relating to the eye and the surrounding area, such as the eyelids 110, 112.

In this exemplary embodiment, the sensor 102 may be at least partially embedded into the ophthalmic device 100. The sensor 102 may be or comprise one or more electrodes configured to sense a capacitance and/or a change in capacitance as the conditions of the eye and/or eyelid change. For example, when various portions of the electrodes comprised by the sensor 102 may be in proximity to the eyelids 110, 112, as illustrated in FIG. 2 and described below. The sensor 102 may be configured to provide a measurable capacitance. As such, when the position of the upper eyelid 110 and/or the lower eyelid 112, relative to the sensor 102, changes, the measurable capacitance may change. Therefore, various capacitance signals may be used to represent positions of the eyelids 110, 112, which may operate as a representation of eye position and/or eye gaze.

The sensor circuit 104 or sensor system may be configured to process signals received by the sensor 102. As an example, the sensor circuit 104 may be configured to amplify a signal to facilitate detection of small changes in signal level. As a further example, the sensor circuit 104 may be configured to amplify a signal to a useable level for the remainder of the system, such as giving a signal enough power to be acquired by various components of the sensor circuit 104 and/or the analog-to-digital converter 106.

In this exemplary embodiment, the analog-to-digital converter 106 may be used to convert an analog signal output from the amplifier into a digital signal for processing. For example, the analog-to-digital converter 106 may convert an analog signal output from the sensor circuit 104 into a digital signal that may be useable by subsequent or downstream circuits, such as a digital signal processing system 108 or microprocessor. A digital signal processing system or digital signal processor 108 may be utilized for digital signal processing, including one or more of filtering, processing, detecting, and otherwise manipulating/processing sampled data to discern a ciliary muscle signal from noise and interference. The digital signal processor 108 may be preprogrammed with the ciliary muscle responses described above. The digital signal processor 108 may be implemented utilizing analog circuitry, digital circuitry, software and/or preferably a combination thereof.

A power source 116 supplies power for numerous components comprising the sensor system. The power may be supplied from a battery, energy harvester, or other suitable means as is known to one of ordinary skill in the art. Essentially, any type of power source may be utilized to provide reliable power for all other components of the system. The system controller 114 may control other aspects of a powered contact lens depending on input from the digital signal processor 108, for example, changing the focus or refractive power of an electronically controlled lens through an actuator 118. Other functions and operations may be controlled by the system controller 114 based on various inputs.

In further alternate exemplary embodiments, the system controller 114 may receive input from sources including one or more of a contact sensor, a blink detector, and a fob control. By way of generalization, it may be obvious to one skilled in the art that the method of activating and/or controlling the system controller 114 may require the use of one or more activation methods. For example, an electronic or powered contact lens may be programmable specific to an individual user, such as programming a lens to recognize both of an individual's ciliary muscle signals when performing various actions, for example, focusing on an object far away, or focusing on an object that is near, and an individual's blink patterns. In some exemplary embodiments, using more than one method to activate an electronic contact lens, such as eyelid position, gaze, and blink detection, may give the ability for each method to crosscheck with another before activation of the contact lens occurs. An advantage of crosschecking may include mitigation of false positives, such as minimizing the chance of unintentionally triggering a lens to activate.

Figure 2 illustrates an ophthalmic device 200, comprising a sensor system, shown on the front surface of the eye 201 or cornea in accordance with another exemplary embodiment of the present disclosure. In this exemplary embodiment, the sensor system may be similar to the components described and shown in FIG. 1. A sensor 202 may be disposed on or in the ophthalmic device 200 and may comprise one or more electrodes 203 configured to provide a measurable capacitance. Accordingly, as an upper eyelid 210 and/or lower eyelid 212 changes position, the eyelids 210, 212 may cause a capacitance in the sensor 202 to fluctuate, thereby indicating a position of the eyelids 210, 212. As the eyelids 210, 212 move to another position, the capacitance at the sensor 202 may change and indicate a new position. As shown in FIG. 2, the electrodes 203 may have a generally curvilinear shape and may be configured in a generally annular configuration about the eye 201. As shown, the sensor 202 comprises four electrodes 203; however, any number of electrodes may be used. As an illustrative example, the electrodes 203 may be spaced from each other. As shown, electrode 203a is disposed between electrode 203b and electrode 203d, opposite electrode 203c. Electrode 203b is disposed between electrode 203a and electrode 203c, opposite electrode 203d. Electrode 203c is disposed between electrode 203b and electrode 203d, opposite electrode 203a. Electrode 203d is disposed between electrode 203a and electrode 203c, opposite electrode 203b. Various configurations may be used. As a further example, the sensor 202 may comprise two electrodes 203 such as electrodes configured in opposite quartile regions of the ophthalmic device 200, such as electrode pair 203a and 203c; or electrode pair 203b and 203d. Various configurations of the electrodes 203 may allow detection of various positions of the eyelids 210, 212, even as the rotation of the ophthalmic device may change relative to the eyelids 201, 212.

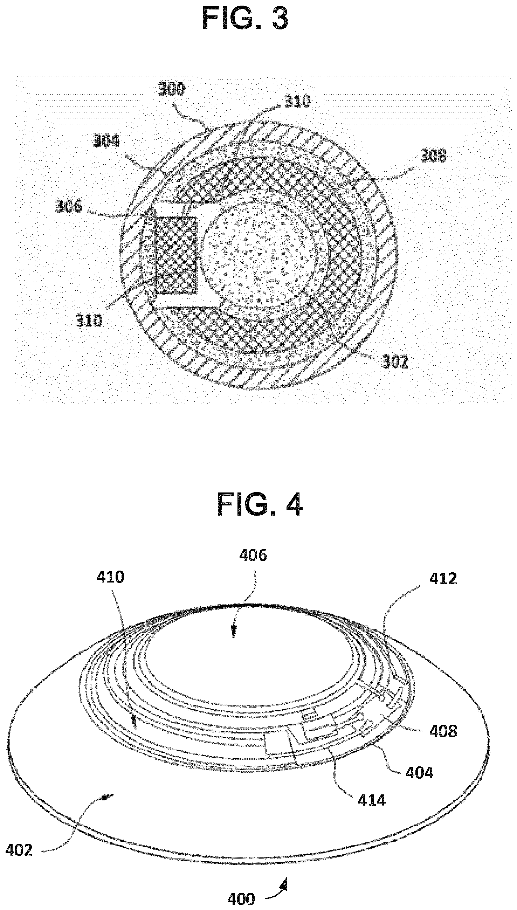

Referring now to Figure 3, there is illustrated, in planar view, a wearable electronic ophthalmic device comprising a sensor in accordance with the present disclosure. The ophthalmic device 300 comprises an optic zone 302 and a peripheral zone 304. The optic zone 302 may function to provide one or more of vision correction, vision enhancement, other vision-related functionality, mechanical support, or even a void to permit clear vision. In accordance with the present disclosure, the optic zone 302 may comprise a variable optic element configured to provide enhanced vision at near and distant ranges based on signals sensed from the ciliary muscle. The variable-optic element may comprise any suitable device for changing the focal length of the lens or the refractive power of the lens based upon activation signals from the sensing system described herein. For example, the variable optic element may be as simple as a piece of optical grade plastic incorporated into the lens with the ability to have its spherical curvature changed. The peripheral zone 304 comprises one or more of electrical circuits 306, a power source 308, electrical interconnects 310, mechanical support, as well as other functional elements.

The electrical circuits 306 may comprise one or more integrated circuit die, printed electronic circuits, electrical interconnects, and/or any other suitable devices, including the sensing circuitry described herein. The power source 308 may comprise one or more of battery, energy harvesting, and or any other suitable energy storage or generation devices. It is readily apparent to the skilled artisan that Figure 3 only represents one exemplary embodiment of an electronic ophthalmic lens and other geometrical arrangements beyond those illustrated may be utilized to optimize area, volume, functionality, runtime, shelf life as well as other design parameters. It is important to note that with any type of variable optic, the fail-safe is distance vision. For example, if power were to be lost or if the electronics fail, the wearer is left with an optic that allows for distance vision.

Figure 4 is a diagrammatic representation of an exemplary electronic insert, including a combined blink detection and communication system, positioned in a powered or electronic ophthalmic device in accordance with the present disclosure. As shown, a contact lens 400 comprises a soft plastic portion 402 which comprises an electronic insert 404. This insert 404 includes a lens 406 which is activated by the electronics, for example, focusing near or far depending on activation. Integrated circuit 408 mounts onto the insert 404 and connects to batteries 410, lens 406, and other components as necessary for the system. The integrated circuit 408 includes a sensor 412 and associated signal path circuits. The sensor 412 may comprise any sensor configuration such as those described herein. The sensor 412 may also be implemented as a separate device mounted on the insert 404 and connected with wiring traces 414.

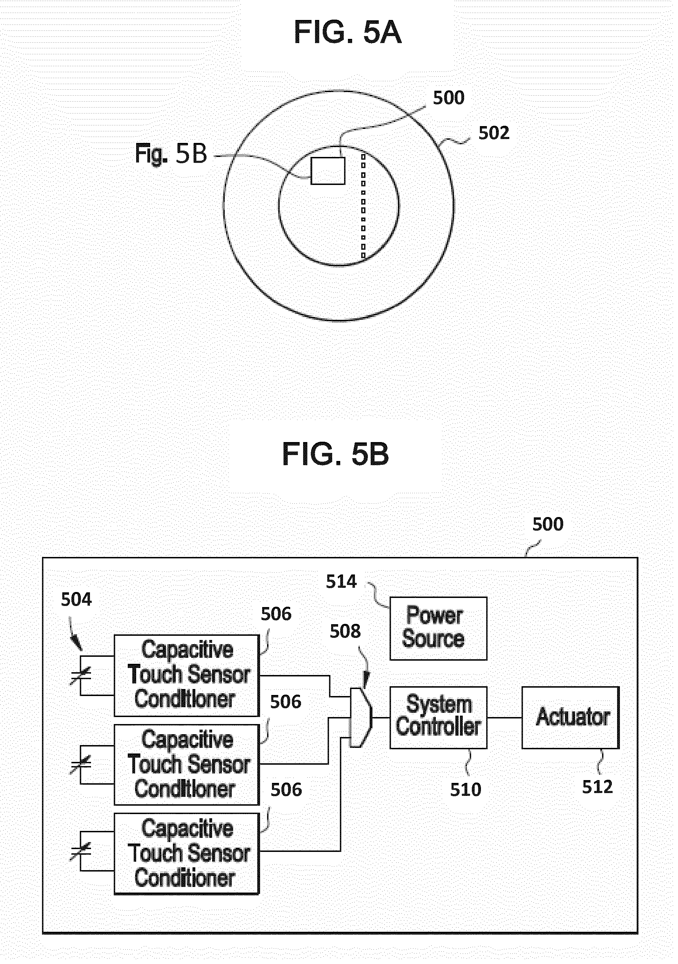

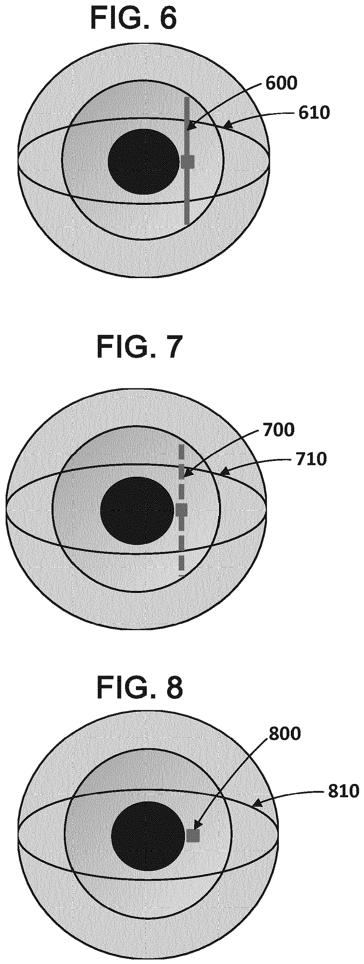

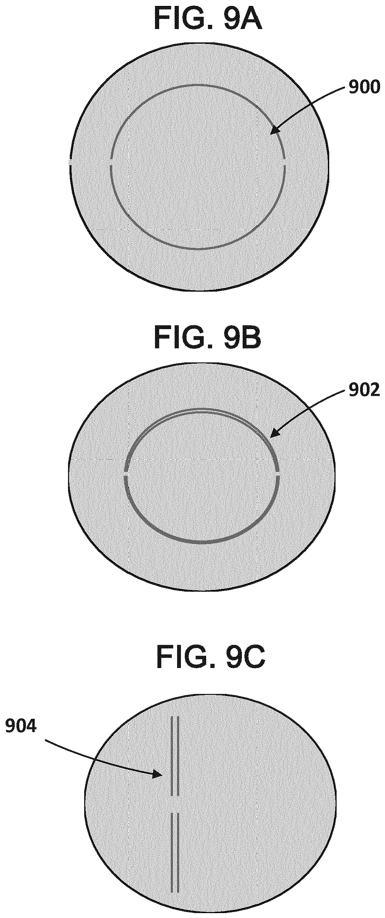

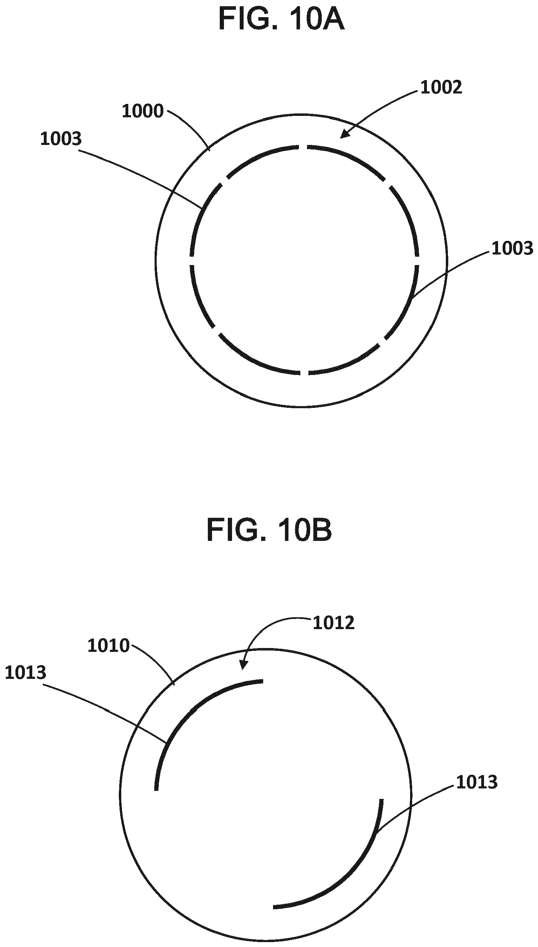

Figures 5A and 5B illustrate an alternate exemplary detection system 500 incorporated into an ophthalmic device 502 such as a contact lens. Figure 5A shows the system 500 on the device 502 and Figure 5B shows an exemplary schematic view of the system 500. In this exemplary embodiment, capacitive touch sensors 504 may be used to sense a capacitance adjacent an eye of the user of the ophthalmic device 502. As an example, the capacitive touch sensors 504 may be configured to detect a capacitance that may be affected by a position of one or more of an upper eyelid and a lower eyelid of the user. As such, the sensed capacitance may be indicative of a position of the eyelid(s) and may represent a gaze or position of the eye. One or more of the capacitive touch sensors 504 may be configured as linear sensor 600 (FIG. 6), a segmented sensor 700 (FIG. 7), and/or an integrating sensor 800 (FIG. 8) configured to integrate a response over a sensor area. In the various configurations illustrated in FIGS. 6-8, the sensors 600, 700, 800 may be configured to sense a capacitance due at least in part to a position of an eyelid 610, 710, 810. Additionally, or alternatively, the sensors 504 may be configured as a dual wire single capacitive sensor 900 (FIG. 9A) and/or a dual wire dual capacitive sensor 902 (FIG. 9B) having a generally curvilinear configuration. Additionally, or alternatively, the sensors 504 may be configured as a dual wire dual capacitive sensor 904 (FIG. 9C) having a generally straight configuration. Additionally, or alternatively, the sensors 504 may be configured in a generally annular configuration such as illustrated in FIG. 2. Any number of sensors may be configured. For example, FIG. 10A illustrates an ophthalmic device 1000 comprising a sensor 1002 having eight traces or electrodes 1003 configured in a generally annular configuration. As a further example, FIG. 10B illustrates an ophthalmic device 1010 comprising a sensor 1012 having two traces or electrodes 1013 configured in a generally annular configuration, wherein each of the electrodes 1013 have a generally curvilinear shape and extend less than half of the circumference of the ophthalmic device 1010.

Returning to Figures 5A and 5B, the capacitive touch sensors 504 may comprise a variable capacitor, which may be implemented in a physical manner such that the capacitance varies with proximity or touch, for example, by implementing a grid covered by a dielectric. Sensor conditioners 506 create an output signal proportional to the capacitance, for example, by measuring the change in an oscillator comprising the variable capacitor or by sensing the ratio of the variable capacitor to a fixed capacitor with a fixed-frequency AC signal. The output of the sensor conditioners 506 may be combined with a multiplexer 508 to reduce downstream circuitry.

In certain embodiments, downstream circuitry may include a system controller 510, which may comprise an analog-to-digital converter (ADC) that may be used to convert a continuous, analog signal into a sampled, digital signal appropriate for further signal processing. For example, the ADC may convert an analog signal into a digital signal that may be useable by subsequent or downstream circuits, such as a digital signal processing system or microprocessor, which may be part of the system controller 510 circuit. A digital signal processing system or digital signal processor may be utilized for digital signal processing, including one or more of filtering, processing, detecting, and otherwise manipulating/processing sampled data. The digital signal processor may be preprogrammed with various lid patterns. As an example, a data store of capacitive measurements or signatures may be mapped to particular positions of an upper eyelid and a lower eyelid. As such, when capacitive measurements matching or near a particular signature are detected, the associated eyelid position(s) may be extrapolated. The digital signal processor also comprises associated memory. The digital signal processor may be implemented utilizing analog circuitry, digital circuitry, software, and/or preferably a combination thereof.

The system controller 510 receives inputs from the capacitance sensor conditioner 506 via a multiplexor 508, for example, by activating each sensor 504 in order and recording the values. It may then compare measured values to pre-programmed patterns and historical samples to determine lid position. It may then activate a function in an actuator 512, for example, causing a variable-focus lens to change to a closer focal distance. The capacitor touch sensors 504 may be laid out in a physical pattern similar to that previously described and shown in references to FIGS. 2 and 6-10, but would be optimized for detecting changes in capacitance with lid position. The sensors 504, and/or the whole electronic system, may be encapsulated and insulated from the saline contact lens environment. As the eyelid covers a sensor 504, the change in capacitance may be detected. Various configurations of the sensors 504 may facilitate optimal sensing conditions as the ophthalmic device 502 shifts or rotates.

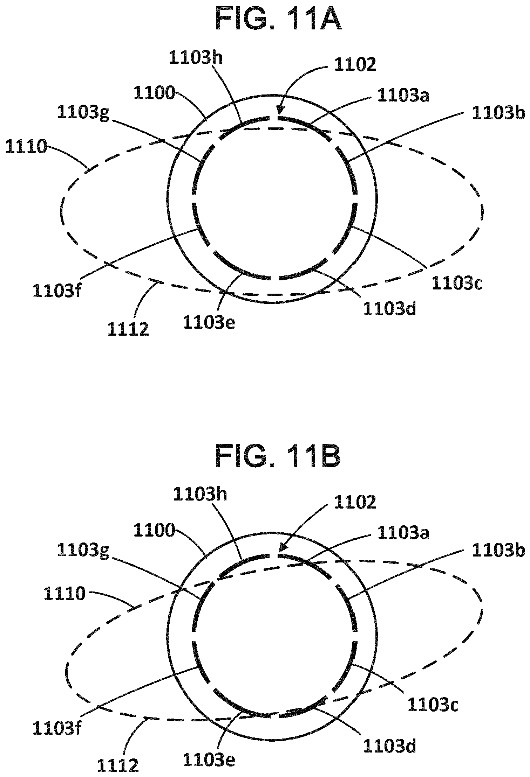

The system controller 510 is preferably preprogrammed to sample each sensor 504 on the eye to detect lid position and provide an appropriate output signal to an actuator 512. The system controller 510 also comprises associated memory. The system controller 510 may combine recent samples of the sensors 504 to preprogrammed patterns correlating to lid open and squinting positions. When the pattern matches that of lid droop associated with near accommodation, for example, the top eyelid drooping, the system controller 510 may trigger the actuator 512 to change focus state of a variable power optic associated with the powered contact lens. Recording a user's eyelid patterns under various capacitance and focal distance situations may be required to program the system controller 510 for reliable detection. The system 500 may need to differentiate between eyelid position changes in various conditions. This differentiation may be accomplished through proper selection of the sampling frequency, amplifier gain, and other system parameters, optimization of sensors placement in the contact lens, determination of lid position patterns, recording capacitance, comparing each sensor 504 to adjacent and all sensors 504, and other techniques to discern lid position uniquely. As an illustrative example, FIGS. 11A-11C illustrate various lid positions as they may overlay traces or electrodes 1103 of a sensor 1102. As the gaze of a user changes, the eyelid position changes and may overlay different portions of the sensor 1102, thereby causing fluctuation in capacitance measurement taken from one or more of the electrodes 1103. Such fluctuation and/or discrete capacitance measurements at sampled times may be mapped to particular eyelid positions. As such, when the same capacitance measurements or fluctuations in the capacitance are detected, the mapped eyelid position may be determined. Additionally, or alternatively, eye gaze may be mapped to particular eyelid position to allow gaze to be determined from a capacitance measurement.

Returning to FIGS. 5A and 5B, a power source 514 supplies power for numerous components comprising the lid position sensor system 500. The power source 514 may also be utilized to supply power to other devices on the contact lens. The power may be supplied from a battery, energy harvester, or other suitable means as is known to one of ordinary skill in the art. Essentially, any type of power source 514 may be utilized to provide reliable power for all other components of the system. A lid position sensor array pattern, processed from analog to digital, may enable activation of the system controller 510 or a portion of the system controller 510. Furthermore, the system controller 510 may control other aspects of a powered contact lens depending on input from the multiplexor 508, for example, changing the focus or refractive power of an electronically controlled lens through the actuator 512.

In one exemplary embodiment, the electronics and electronic interconnections are made in the peripheral zone of a contact lens rather than in the optic zone. In accordance with an alternate exemplary embodiment, it is important to note that the positioning of the electronics need not be limited to the peripheral zone of the contact lens. All of the electronic components described herein may be fabricated utilizing thin film technology and/or transparent materials. If these technologies are utilized, the electronic components may be placed in any suitable location as long as they are compatible with the optics. The activities of the digital signal processing block and system controller (system controller 510 in Figure 5B) depend on the available sensor inputs, the environment, and user reactions. The inputs, reactions, and decision thresholds may be determined from one or more of ophthalmic research, preprogramming, training, and adaptive/learning algorithms. For example, the general characteristics of eyelid movement may be well-documented in literature, applicable to a broad population of users, and pre-programmed into system controller. However, an individual's deviations from the general expected response may be recorded in a training session or part of an adaptive/learning algorithm which continues to refine the response in operation of the electronic ophthalmic device. In one exemplary embodiment, the user may train the device by activating a handheld fob, which communicates with the device, when the user desires near focus. A learning algorithm in the device may then reference sensor inputs in memory before and after the fob signal to refine internal decision algorithms. This training period could last for one day, after which the device would operate autonomously with only sensor inputs and not require the fob.

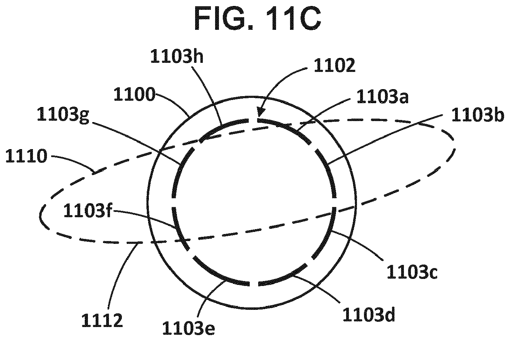

Figures 11A, 11B, and 11C illustrate an ophthalmic device 1100, comprising a sensor system, that may be placed adjacent an eye or cornea of a user in accordance with another exemplary embodiment of the present disclosure. In this exemplary embodiment, the sensor system may be similar to the components described and shown in FIG. 1. The sensor 1102 may be disposed on or in the ophthalmic device 1100 and may comprise one or more electrodes 1103a-h configured to provide a measurable capacitance. Accordingly, as an upper eyelid 1110 and/or lower eyelid 1112 changes position, the eyelids 1110, 1112 may cause a capacitance in the sensor 1102 to fluctuate, thereby indicating a position of the eyelids 1110, 1112. As the eyelids 1110, 1112 move to another position, the capacitance at the sensor 1102 may change and indicate a new position. As shown in FIGS. 11A-11C, the electrodes 1103 may have a generally curvilinear shape and may be configured in a generally annular configuration about the ophthalmic device 1100. As shown, the sensor 1102 comprises eight electrodes 1103a-h; however, any number of electrodes may be used. As an illustrative example, the electrodes 1103a-h may be spaced from each other. As shown, electrode 1103a is disposed between electrode 1103b and electrode 1103h, opposite electrode 203e. Electrode 1103b is disposed between electrode 1103a and electrode 1103c, opposite electrode 1103f. Electrode 1103c is disposed between electrode 1103b and electrode 1103d, opposite electrode 1103g. Electrode 1103d is disposed between electrode 1103c and electrode 1103e, opposite electrode 1103h. Electrode 1103e is disposed between electrode 1103d and electrode 1103f, opposite electrode 1103a. Electrode 1103f is disposed between electrode 1103e and electrode 1103g, opposite electrode 1103b. Electrode 1103g is disposed between electrode 1103f and electrode 1103h, opposite electrode 1103c. Electrode 1103h is disposed between electrode 1103g and electrode 1103a, opposite electrode 1103d. Various configurations may be used. As a further example, the sensor 1102 may comprise two electrodes 1103 such as electrodes configured in opposite quartile regions of the ophthalmic device 1100, such as electrode pair 1103a and 1103e; or electrode pair 1103g and 1103c. Various configurations of the electrodes 1103a-h may allow detection of various positions of the eyelids 1110, 1112, even as the rotation of the ophthalmic device may change relative to the eyelids 1101, 1112.

Figures 11A, 11B, and 11C illustrate various positions of the eyelids 1110, 1112 as they may overlay the electrodes 1103 of a sensor 1102. As the gaze of a user changes, the position of the eyelids 1110, 1112 changes and may overlay different portions of the sensor 1102, thereby causing fluctuation in capacitance measurement taken from one or more of the electrodes 1103.

As shown in FIG. 11A, a gaze angle may be taken as a zero degree down gaze, where the upper eyelid 1110 overlays electrode 1103a and electrode 1103h and the lower eyelid 1112 overlays none of the electrodes 1103a-h. As such, the capacitance measurement at each of the electrodes 1103a-h may provide a capacitive sensing signature representative of the zero degree down gaze. In particular, electrode 1103a and electrode 1103h may detect a capacitance measurement indicative of the overlaying upper eyelid 1110. This information may be stored, for example, via a system controller 114 (FIG. 1) and may be referenced subsequently. As an example, when a subsequent capacitance measurement is found to be the same or similar to the stored measurement, the positions of the eyelids 1110, 1112 may be determined. Additionally, or alternatively, the eye gaze may be determined. As a further example, the stored measurements may represent the activation or deactivation of a particular electrode 1103 have a sensed capacitance over a preset threshold. For example, the stored measurements may indicate the for a zero degree down gaze, the electrodes 1103a, 1103h will be activated, but the other electrodes 1103b-1103g will be deactivated. Capacitance measurements may be absolute, binary, actual, and/or conditioned in various manners.

As shown in FIG. 11B, a gaze angle may be taken as a twenty-five degree down gaze, where the upper eyelid 1110 overlays electrode 1103a and electrode 1103h and the lower eyelid 1112 overlays electrode 1103d and electrode 1103e. As such, the capacitance measurement at each of the electrodes 1103a-h may provide a capacitive sensing signature representative of the twenty-five degree down gaze. In particular, electrode 1103a and electrode 1103h may detect a capacitance measurement indicative of the overlaying upper eyelid 1110. Electrode 1103d and electrode 1103e may detect a capacitance measurement indicative of the overlaying lower eyelid 1112. This information may be stored, for example, via a system controller 114 (FIG. 1) and may be referenced subsequently. As an example, when a subsequent capacitance measurement is found to be the same or similar to the stored measurement, the positions of the eyelids 1110, 1112 may be determined. Additionally, or alternatively, the eye gaze may be determined. As a further example, the stored measurements may represent the activation or deactivation of a particular electrode 1103 have a sensed capacitance over a preset threshold. For example, the stored measurements may indicate the for a zero degree down gaze, the electrodes 1103a, 1103d, 1103e, 1103h will be activated, but the other electrodes will be deactivated. Capacitance measurements may be absolute, binary, actual, and/or conditioned in various manners.

As shown in FIG. 11C, a gaze angle may be taken as a forty-five degree down gaze, where the upper eyelid 1110 overlays electrode 1103a and electrode 1103h and the lower eyelid 1112 overlays electrode 1103c and electrode 1103f. As such, the capacitance measurement at each of the electrodes 1103a-h may provide a capacitive sensing signature representative of the forty-five degree down gaze. In particular, electrode 1103a and electrode 1103h may detect a capacitance measurement indicative of the overlaying upper eyelid 1110. Electrode 1103c and electrode 1103f may detect a capacitance measurement indicative of the overlaying lower eyelid 1112. This information may be stored, for example, via a system controller 114 (FIG. 1) and may be referenced subsequently. As an example, when a subsequent capacitance measurement is found to be the same or similar to the stored measurement, the positions of the eyelids 1110, 1112 may be determined. Additionally, or alternatively, the eye gaze may be determined. As a further example, the stored measurements may represent the activation or deactivation of a particular electrode 1103 have a sensed capacitance over a preset threshold. For example, the stored measurements may indicate the for a zero degree down gaze, the electrodes 1103a, 1103c, 1103f, 1103h will be activated, but the other electrodes will be deactivated. Capacitance measurements may be absolute, binary, actual, and/or conditioned in various manners.

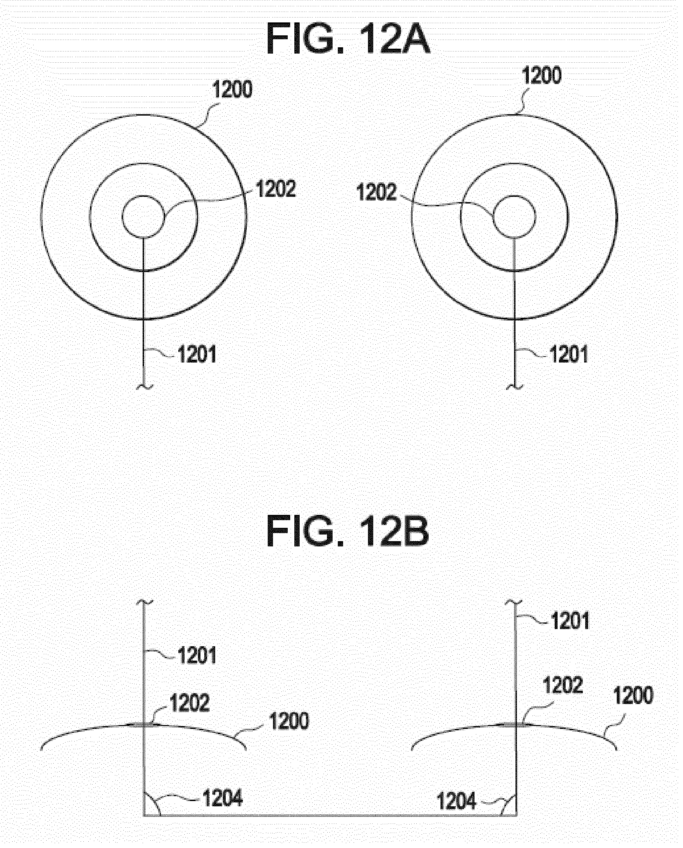

Figures 12A and 12B illustrate different views of the two eyes 1200 of an individual who is gazing at a distant object which requires far focus, for example, driving a car, instead of near focus, for example, reading a book. Figure 12A illustrates a front perspective of the eyes 1200, whereas Figure 12B illustrates a top perspective of the eyes 1200. While gazing at a distant object, not illustrated, the pupils 1202 are centered and track together. Lines 1201 between the pupils 1202 and the object under observation are parallel as is shown by angles 1204 both being ninety (90) degrees. This is because the distance between the two eyes 1200 on any individual is much less than the distance from the eyes 1200 to the object under observation. As an individual tracks the movement of a distant object, although the eyes 1200 move, the angles 1204 remain very close to ninety (90) degrees, again because the distance between the two eyes 1200 is much less than the distance from the eyes 1200 to the object under observation.

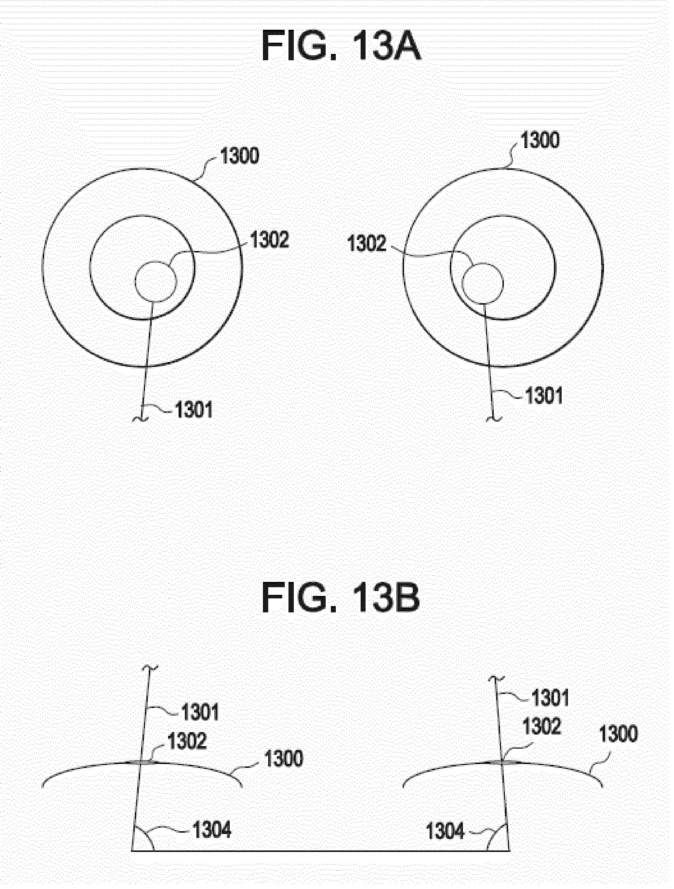

Figures 13A and 13B illustrate a pair of eyes 1300 substantially similar to those illustrated in Figures 12A and 12B, with the exception that in this example the object under observation, not illustrated, is close or near rather than distant. Since the distance between the eyes 1300 is now appreciable relative to the distance from the eyes 1300 to the object under observation, the eyes 1300 converge to keep the object under observation within the field of view. As illustrated, via exaggeration, pupils 1302 converge and move closer together. Lines 1301 drawn between the pupils and the object under observation are no longer parallel, and the angles 1304 are less than ninety (90) degrees. This phenomena may be easily observed by having a subject first focus on his or her finger at an approximate distance of two (2) feet with his or her arm fully extended. As the subject brings his or her finger closer, his or her eyes will converge towards his or her nose becoming "cross-eyed."

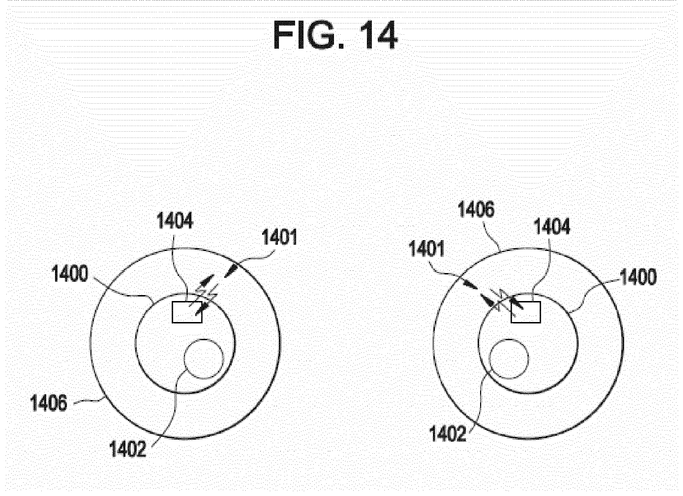

Figure 14 illustrates a system by which the convergence described with respect to Figures 12A, 12B, 13A and 13B may be sensed and communicated between a pair of contact lenses 1400. Pupils 1402 are illustrated converged for near object viewing. Pupil position and convergence detection systems 1404 incorporated within contact lenses 1400 that are positioned on eyes 1406 track the position of the pupils 1402 and/or the contact lenses 1400, for example, with reverse-facing photodetectors to observe the pupils 1402 or with accelerometers to track movement of the eyes 1406 and hence the pupils 1402. The pupil position and convergence detector systems 1404 may comprise several components forming a more complex system, for example a 3-axis accelerometer, signal conditioning circuitry, a controller, memory, power supply, and a transceiver as is described in detail subsequently. Communication channel 1401 between the two contact lenses 1400 allows the pupil position and convergence detection systems 1404 to synchronize on pupil position. Communication may also take place with an external device, for example, spectacle glasses or a smartphone. Communication between the contact lenses 1400 is important to detect convergence. For example, without knowing the position of both pupils 1402, simply gazing down to the left may be detected as convergence by the right eye since the pupil 1402 has similar movement for both actions. However, if the right pupil is detected moving down to the left while the pupil of the left eye is detected moving down to the right, convergence may be construed. Communication between the two contact lenses 1400 may take the form of absolute or relative position, or may simply be a "convergence suspected" signal if the eye moves in the expected direction of convergence. In this case, if a given contact lens detects convergence itself and receives a convergence indication from the adjacent contact lens, it may activate a change in stage, for example, switching a variable-focus or variable power optic equipped contact lens to the near distance state to support reading. Other information useful for determining the desire to accommodate (focus near), for example, lid position and ciliary muscle activity, may also be transmitted over the communication channel 1401 if the contact lenses are so equipped. It should also be appreciated that communication over the channel 1401 could comprise other signals sensed, detected, or determined by each lens 1406 and 15 used for a variety of purposes, including vision correction, vision enhancement, entertainment, and novelty.

In accordance with one exemplary embodiment, a digital communication system comprises a number of elements which when implemented, may take on any number of forms. The digital communication system generally comprises an information source, a source encoder, a channel encoder, a digital modulator, a channel, a digital demodulator, a channel decoder and a source decoder. The information source may comprise any device that generates information and/or data that is required by another device or system. The source may be analog or digital. If the source is analog, its output is converted into a digital signal comprising a binary string. The source encoder implements a process of efficiently converting the signal from the source into a sequence of binary digits. The information from the source encoder is then passed into a channel encoder where redundancy is introduced into the binary information sequence. This redundancy may be utilized at the receiver to overcome the effects of noise, interference and the like encountered on the channel. The binary sequence is then passed to a digital modulator which in turn converts the sequence into analog electrical signals for transmission over the channel. Essentially, the digital modulator maps the binary sequences into signal waveforms or symbols. Each symbol may represent the value of one or more bits. The digital modulator may modulate a phase, frequency or amplitude of a high frequency carrier signal appropriate for transmission over or through the channel. The channel is the medium through which the waveforms travel, and the channel may introduce interference or other corruption of the waveforms. In the case of the wireless communication system, the channel is the atmosphere. The digital demodulator receives the channel-corrupted waveform, processes it and reduces the waveform to a sequence of numbers that represent, as nearly as possible, the transmitted data symbols. The channel decoder reconstructs the original information sequence from knowledge of the code utilized by the channel encoder and the redundancy in the received data. The source decoder decodes the sequence from knowledge of the encoding algorithm, wherein the output thereof is representative of the source information signal. It is important to note that the above described elements may be realized in hardware, in software or in a combination of hardware and software. In addition, the communication channel may comprise any type of channel, including wired and wireless. In wireless, the channel may be configured for high frequency electromagnetic signals, low frequency electromagnetic signals, visible light signals and infrared light signals.

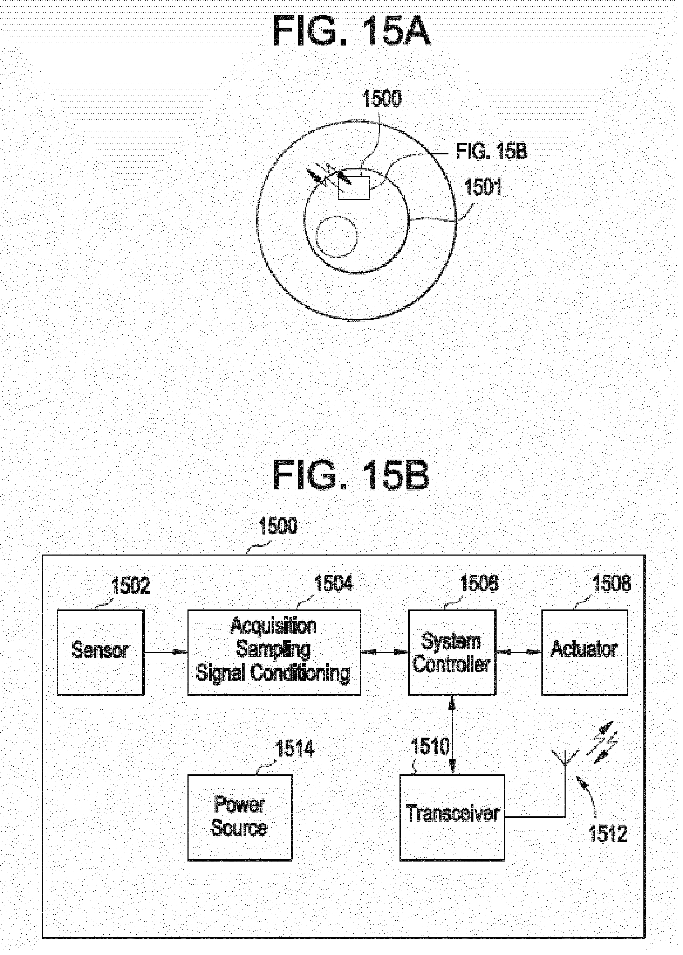

Figures 15A and B are diagrammatic representations of an exemplary pupil position and convergence detection system 1500 for control of one or more aspects of a powered ophthalmic lens. Sensor 1502 detects the movement and/or position of the pupil or, more generally, the eye. The sensor 1502 may be implemented as a multi-axis accelerometer on a contact lens 1501. With the contact lens 1501 being affixed to the eye and generally moving with the eye, an accelerometer on the contact lens 1501 may track eye movement. The sensor 1502 may also be implemented as a rear-facing camera or sensor which detects changes in images, patterns, or contrast to track eye movement. Alternately, the sensor 1502 may comprise neuromuscular sensors to detect nerve and/or muscle activity which moves the eye in the socket. There are six muscles attached to each eye globe which provide each eye with a full range of movement and each muscle has its own unique action or actions. These six muscles are innervated by one of the three cranial nerves. It is important to note that any suitable device may be utilized as the sensor 1502, and more than a single sensor 1502 may be utilized. The output of the sensor 1502 is acquired, sampled, and conditioned by signal processor 1504. The signal processor 1504 may include any number of devices including an amplifier, a transimpedance amplifier, an analog-to-digital converter, a filter, a digital signal processor, and related circuitry to receive data from the sensor 1502 and generate output in a suitable format for the remainder of the components of the system 1500. The signal processor 1504 may be implemented utilizing analog circuitry, digital circuitry, software, and/or preferably a combination thereof. It should be appreciated that the signal processor 1504 is co-designed with the sensor 1502 utilizing methods that are known in the relevant art, for example, circuitry for acquisition and conditioning of an accelerometer are different than the circuitry for a muscle activity sensor or optical pupil tracker. The output of the signal processor 1504 is preferentially a sampled digital stream and may include absolute or relative position, movement, detected gaze in agreement with convergence, or other data. System controller 1506 receives input from the signal processor 1504 and uses this information, in conjunction with other inputs, to control the electronic contact lens 1501. For example, the system controller 1506 may output a signal to an actuator 1508 that controls a variable power optic in the contact lens 1501. If, for example, the contact lens 1501 is currently in a far focus state and the sensor 1502 detects convergence, the system controller 1506 may command the actuator 1508 to change to a near focus state. System controller 1506 may both trigger the activity of sensor 1502 and the signal processor 1504 while receiving output from them. A transceiver 1510 receives and/or transmits communication through antenna 1502. This communication may come from an adjacent contact lens, spectacle lenses, or other devices. The transceiver 1510 may be configured for two way communication with the system controller 1506. Transceiver 1510 may contain filtering, amplification, detection, and processing circuitry as is common in transceivers. The specific details of the transceiver 1510 are tailored for an electronic or powered contact lens, for example the communication may be at the appropriate frequency, amplitude, and format for reliable communication between eyes, low power consumption, and to meet regulatory requirements. Transceiver 1510 and antenna 1512 may work in the radio frequency (RF) bands, for example 2.4 GHz, or may use light for communication. Information received from transceiver 1510 is input to the system controller 1506, for example, information from an adjacent lens which indicates convergence or divergence. System controller 1506 uses input data from the signal processor 1504 and/or transceiver 1510 to decide if a change in system state is required. The system controller 1506 may also transmit data to the transceiver 1510, which then transmits data over the communication link via antenna 1512. The system controller 1506 may be implemented as a state machine, on a field-programmable gate array, in a microcontroller, or in any other suitable device. Power for the system 1500 and components described herein is supplied by a power source 1514, which may include a battery, energy harvester, or similar device as is known to one of ordinary skill in the art. The power source 1514 may also be utilized to supply power to other devices on the contact lens 1501. The exemplary pupil position and convergence detection system 1500 of the present disclosure is incorporated and/or otherwise encapsulated and insulated from the saline contact lens 1501 environment.

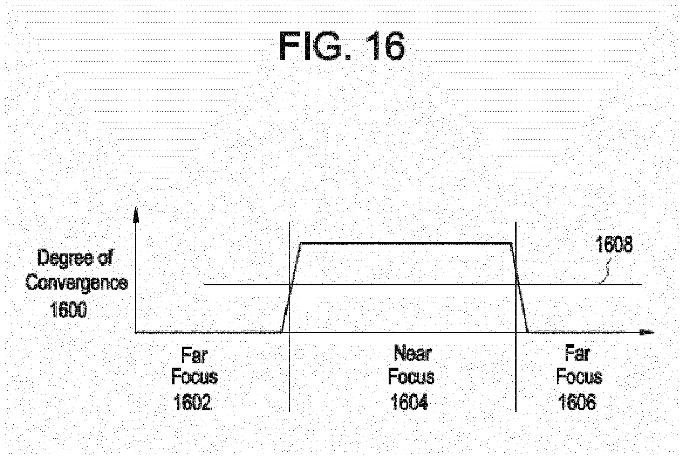

Figure 16 illustrates an exemplary, simplified correlation between convergence 1600 and focal length states 1602, 1604, and 1606 as is commonly documented in the ophthalmic literature. When in the far focus state 1602 and 1606, as described with respect to Figures 12A and 12B, the degree of convergence is low. When in the near focus state 1604, as described with respect to Figures 13A and 13B, the degree of convergence is high. A threshold 1608 may be set in the system controller (element 1506 of Figure 15) to change the state of the electronic ophthalmic lens, for example, focusing a variable optic with add power when the threshold is passed going positive then focusing the variable optic with no add power when the threshold is passed going negative.

Eye tracking is the process of determining either or both where an individual is looking, point of gaze, or the motion of an eye relative to the head. An individual's gaze direction is determined by the orientation of the head and the orientation of the eyes. More specifically, the orientation of an individual's head determines the overall direction of the gaze while the orientation of the individual's eyes determines the exact gaze direction which in turn is limited by the orientation of the head. Information of where an individual is gazing provides the ability to determine the individual's focus of attention and this information may be utilized in any number of disciplines or application, including cognitive science, psychology, human-computer interaction, marketing research and medical research. For example, eye gaze direction may be utilized as a direct input into a controller or computer to control another action. In other words, simple eye movements may be utilized to control the actions of other devices, including highly complex functions. Simple eye movements may be utilized in a manner similar to "swipes" which have become common in touch-screen and smartphone applications, for example, swiping to unlock a device, change applications, change pages, zoom in or out and the like. Eye gaze tracking systems are presently utilized to restore communication and functionality to those who are paralyzed, for example, using eye movements to operate computers. Eye tracking or gaze tracking may also be utilized in any number of commercial applications, for example, what individuals are paying attention to when they are watching television, browsing websites and the like. The data collected from this tracking may be statistically analyzed to provide evidence of specific visual patterns. Accordingly, information garnered from detecting eye or pupil movement may be utilized in a wide range applications. Once again, it is also important to note that the sensed data, in addition to or in alternate use may simply be utilized as part of a collection process rather than as a triggering event. For example, the sensed data may be collected, logged and utilized in treating medical conditions. In other words, it should also be appreciated that a device utilizing such a sensor may not change state in a manner visible to the user; rather the device may simply log data. For example, such a sensor could be used to determine if a user has the proper iris response throughout a day or if a problematic medical condition exists. It is important to note, that eye tracking in accordance with the present disclosure may be set up for gross or fine tracking monitoring. There are a number of currently available devices for tracking eye movement, including video-based eye trackers, search coils and arrangements for generating electrooculograms. Search coils or inductive sensors are devices which measure the variations of the surrounding magnetic fields. Essentially, a number of coils may be imbedded into a contact lens type device and the polarity and amplitude of the current generated in the coils varies with the direction and angular displacement of the eye. An electrooculogram is generated by a device for the detection of eye movement and eye position based on the difference in electrical potential between electrodes placed on either side of the eye. All of these devices are not suitable for use with a wearable, comfortable electronic ophthalmic lens or powered contact lens. Therefore, in accordance with another exemplary embodiment, the present disclosure is directed to a powered contact lens comprising a gaze sensor incorporated directly into the contact lens. As described, the gaze sensor may comprise a capacitive sensor configured to detect capacitance based on the overlay of an upper eyelid and a lower eyelid on capacitive sensor electrodes. As such, the eyelid positions may be determined based on the capacitance measurements of the electrodes. As an example, a number of capacitance measurements may be sampled and associated with known eyelid positions. As such, when the same or similar capacitance measurements are detected, the eyelid positions may be determined from the associated positions and sampled capacitance.

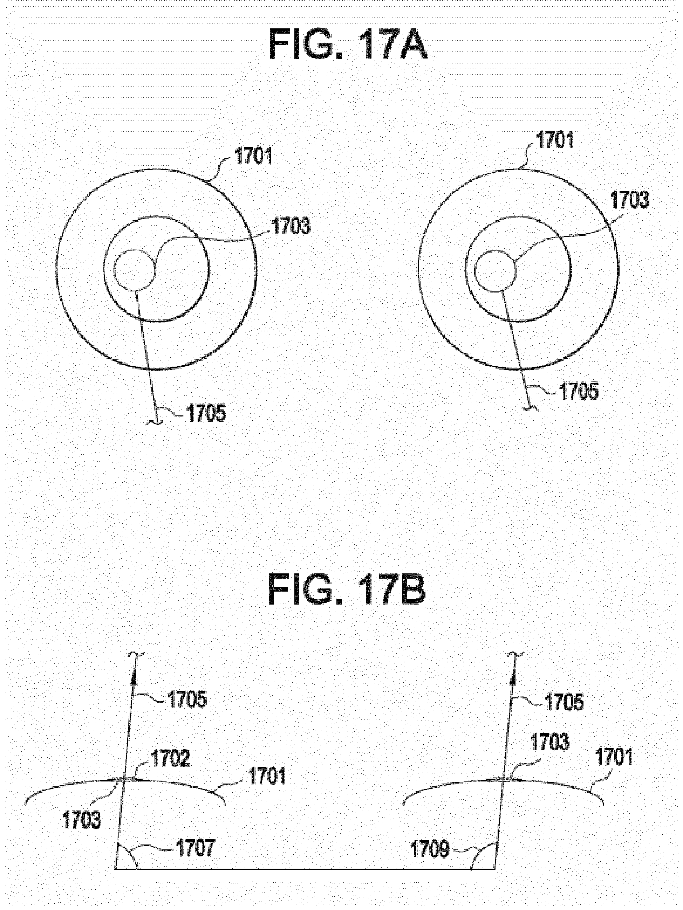

Figures 17A and 17B illustrate a pair of eyes 1701 substantially similar to those 25 illustrated in Figures 12A and 12B, with the exception that in this example the object under observation, not illustrated, is to the right of the user. Figure 17A illustrates a front perspective of the eyes 1701, whereas Figure 17B illustrates a top perspective of the eyes 1701. The position to the right is used for illustrative purposes, but it should be appreciated that the object under observation could be at any visible point in three dimension space with the corresponding changes in eye gaze. As illustrated, via exaggeration, pupils 1703 both face toward the right. Lines 1705 drawn between the pupils 1703 and the object under observation are almost parallel since the object is illustrated to be much farther from the eyes 1701 than the distance between the eyes 1701. Angle 1707 is less than ninety (90) degrees whereas angle 1709 is greater than ninety (90) degrees. These angles are in contrast to previous figures where the angles were either both ninety (90) degrees, when gazing straight ahead at a distant object, or both less than ninety degrees, when gazing straight ahead at a nearby object. As is illustrated in two dimensions, the angle may be used to determine gaze position or, more generally, samples of eye movement may be utilized to determine absolute and relative position and movement of eye gaze.

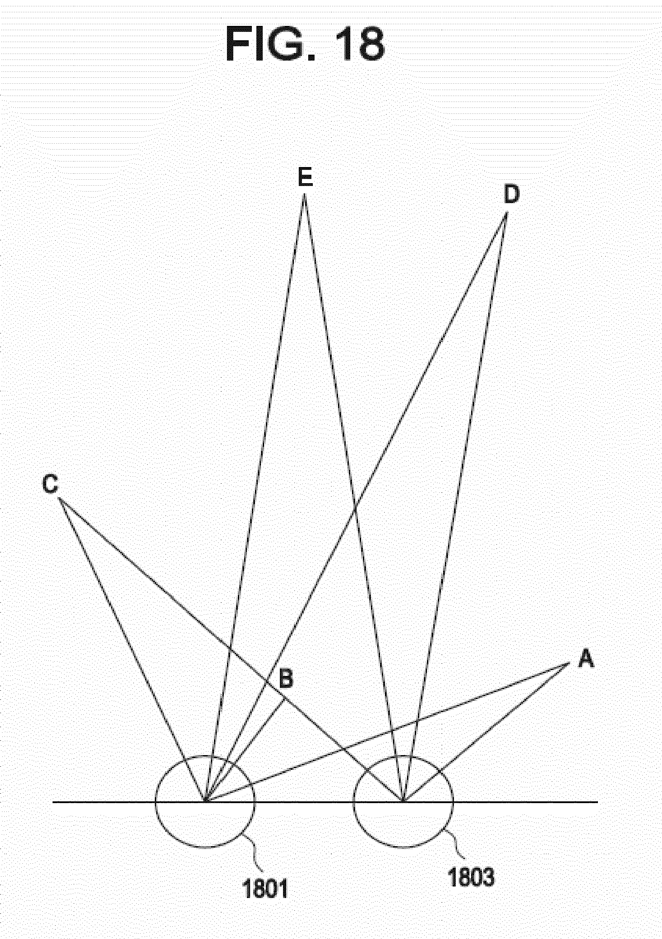

Figure 18 illustrates the geometric systems associated with various gaze directions. Figure 18 is a top view. Eyes 1801 and 1803 are shown gazing upon various targets labeled A, B, C, D, and E. A line connects each eye 1801 and 1803 to each 15 target. A triangle is formed by each of the two lines connecting the eyes 1801 and 1803 with a given target in addition to a line connecting both eyes 1801 and 1803. As may be seen in the illustration, the angles between the direction of gaze in each eye 1801 and 1803 and the line between the two eyes 1801 and 1803 varies for each target. These angles may be measured by the sensor system, determined from indirect sensor measurements, or may only be shown for illustrative purposes. Although shown in two dimensional space for simplicity of illustration, it should be apparent that gaze occurs in three-dimensional space with the corresponding addition of an additional axis. Targets A and B are shown relatively near to the eyes 1801 and 1803, for example, to be read with near-focus accommodation. Target A is to the right of both eyes 1801 and 1803, hence both eyes 1801 and 1803 are pointing right. Measuring the angle formed anticlockwise between the horizontal axis, illustrated collinear with the line connecting the two eyes 1801 and 1803, and direction of gaze, both angles are acute for target A. Now referring to target B the eyes 1801 and 1803 are converged on a target in front of and between both eyes 1801 and 1803. Hence the angle, previously defined as anticlockwise from the horizontal axis and the direction of gaze, is obtuse for the right eye 1803 and acute for the left eye 1801. A suitable sensor system will differentiate the positional difference between targets A and B with suitable accuracy for the application of concern. Target C is shown at intermediate distance for the special case of the right eye 1803 having the same direction of gaze and angle as target B. The gaze direction varies between targets B and C allowing a gaze direction determination system using inputs from both eyes 1801 and 1803 to determine the direction of gaze. Further, a case could be illustrated where another target F lies above target B in three-dimensional space. In such an example, projected into the two-dimensional illustration shown in

Figure 18, the angles from the horizontal axis would be identical to those illustrated for target B. However, the angles normal to the page extending in three-dimensional space would not be equal between the targets. Finally, targets D and E are shown as distant objects. These examples illustrate that as the object under gaze is farther away, the angular difference at the eyes 1801 and 1803 between distant points becomes smaller. A suitable system for detecting gaze direction would have sufficient accuracy to 15 differentiate between small, distant objects.

The direction of gaze may be determined by any number of suitable devices, for example, with reverse-facing photodetectors to observe the pupils or with accelerometers to tack the movement of the eyes. Neuromuscular sensors may also be utilized. By monitoring the six muscles that control eye movement, the precise direction of gaze may be determined. A memory element to store prior position and/or acceleration may be required in addition to a position computation system considering present and past sensor inputs. In addition, the system illustrated in Figures 15A and 15B are equally applicable to the gaze and tracking system of the present disclosure.

The system is preferably programmed to account for gazing geometries in three dimensional space. It is known in the art of optometry that the eyes do not remain completely stable when gazing at a stationary object. Rather, the eyes quickly move back and forth. A suitable system for detecting gaze position would include the necessary filtering and/or compensation to account for visual physiology. For example, such a system may include a low-pass filter or an algorithm specially tuned to a user's natural eye behaviors.

In one exemplary embodiment, the electronics and electronic interconnections are made in the peripheral zone of a contact lens rather than in the optic zone. In accordance with an alternate exemplary embodiment, it is important to note that the positioning of the electronics need not be limited to the peripheral zone of the contact lens. All of the electronic components described herein may be fabricated utilizing thin film technology and/or transparent materials. If these technologies are utilized, the electronic components may be placed in any suitable location as long as they are compatible with the optics.

The activities of the acquisition sampling signal processing block and system controller (1504 and 1506 in Figure 15B, respectively) depend on the available sensor inputs, the environment, and user reactions. The inputs, reactions, and decision thresholds may be determined from one or more of ophthalmic research, preprogramming, training, and adaptive/learning algorithms. For example, the general characteristics of eye movement may be well-documented in literature, applicable to a broad population of users, and pre-programmed into system controller. However, an individual's deviations from the general expected response may be recorded in a training session or part of an adaptive/learning algorithm which continues to refine the response in operation of the electronic ophthalmic device. In one exemplary embodiment, the user may train the device by activating a handheld fob, which communicates with the device, when the user desires near focus. A learning algorithm in the device may then reference sensor inputs in memory before and after the fob signal to refine internal decision algorithms. This training period could last for one day, after which the device would operate autonomously with only sensor inputs and not require the fob. An intraocular lens or IOL is a lens that is implanted in the eye and replaces the crystalline lens. It may be utilized for individuals with cataracts or simply to treat various refractive errors. An IOL typically comprises a small plastic lens with plastic side struts called haptics to hold the lens in position within the capsular bag in the eye. Any of the electronics and/or components described herein may be incorporated into IOLs in a manner similar to that of contact lenses.

Although shown and described in what is believed to be the most practical and preferred embodiments, it is apparent that departures from specific designs and methods described and shown will suggest themselves to those skilled in the art and may be used without departing from the spirit and scope of the disclosure. The present disclosure is not restricted to the particular constructions described and illustrated, but should be constructed to cohere with all modifications that may fall within the scope of the appended claims.

The following is a non-exhaustive list of numbered embodiments which may be claimed:
1. An eyelid position sensing system for a powered ophthalmic device, the eyelid position sensing system comprising:
   a first electrode configured to be selectively overlaid by one or more of an upper eyelid and a lower eyelid of a user;
   a second electrode configured to be selectively overlaid by one or more of an upper eyelid and a lower eyelid of a user; and
   a system controller cooperatively associated with the first electrode and the second electrode to receive a capacitance measurement therefrom, the system controller configured to determine a position of one or more of the upper eyelid and the lower eyelid in spatial coordinates based on the capacitance measurement received from the first electrode and the second electrode.
2. The eyelid position sensing system according to embodiment 1, wherein the first electrode and the second electrode are spaced apart.
3. The eyelid position sensing system according to embodiment 1, wherein the first electrode and the second electrode are disposed on the powered ophthalmic device.
4. The eyelid position sensing system according to embodiment 3, wherein the powered ophthalmic device comprises a contact lens, an intraocular lens, an overlay lens, an ocular insert, or an optical insert, or a combination thereof.
5. The eyelid position sensing system according to embodiment 1, wherein one or more of the first electrode and the second electrode is configured in a generally curvilinear shape.
6. The eyelid position sensing system according to embodiment 1, wherein the first electrode and the second electrode are configured in a generally annular shape.
7. The eyelid position sensing system according to embodiment 1, wherein the first electrode and the second electrode are disposed on opposite sides of a pupil of an eye of the user of the powered ophthalmic device.
8. The eyelid position sensing system according to embodiment 1, further comprising a signal processor configured to receive signals from the sensor, perform digital signal processing, and output one or more to the system controller.
9. The eyelid position sensing system according to embodiment 8, wherein signal processor comprises associated memory.
10. The eyelid position sensing system according to embodiment 1, further comprising a power supply.
11. The eyelid position sensing system according to embodiment 1, wherein the spatial coordinates are in two dimensions.
12. The eyelid position sensing system according to embodiment 1, wherein the spatial coordinates are in three dimensions.
13. The eyelid position sensing system according to embodiment 1, further comprising a transceiver configured to wirelessly interfaces with an external device.
14. A powered ophthalmic device comprising:
   a lens including an optic zone and a peripheral zone; and
   an eye gaze tracking system incorporated into the peripheral zone of the contact lens, the eye gaze tracking system including a capacitive touch sensor to detect a capacitance based at least on a position of one or more of an upper eyelid and a lower eyelid, a system controller cooperatively associated with the capacitive touch sensor, the system controller configured to determine gaze direction in spatial coordinates based on information received from the sensor, and at least one actuator configured to receive the output control signal and implement a predetermined function.
15. The powered ophthalmic device according to embodiment 14, wherein the capacitive touch sensor comprises a plurality of spaced-apart electrodes.
16. The powered ophthalmic device according to embodiment 15, wherein the plurality of spaced-apart electrodes are configured in a generally curvilinear shape.
17. The powered ophthalmic device according to embodiment 15, wherein the plurality of spaced-apart electrodes are configured in a generally annular shape.
18. The powered ophthalmic device according to embodiment 15, wherein at least two of the plurality of spaced-apart electrodes are disposed on opposite sides of a pupil of an eye of the user of the powered ophthalmic device.
19. The powered ophthalmic lens according to embodiment 14, wherein the eye gaze tracking system further comprises a signal processor configured to receive signals from the sensor, perform digital signal processing, and output one or more to the system controller.
20. The powered ophthalmic lens according to embodiment 19, wherein signal processor comprises associated memory.
21. The powered ophthalmic lens according to embodiment 14, wherein the eye gaze tracking system further comprises a power supply.
22. The powered ophthalmic lens according to embodiment 14, wherein the eye gaze tracking system further comprises a communication system for communication with at least a second lens.
23. The powered ophthalmic lens according to embodiment 14, wherein the spatial coordinates are in two dimensions.
24. The powered ophthalmic lens according to embodiment 14, wherein the spatial coordinates are in three dimensions.
25. The powered ophthalmic lens according to embodiment 14, wherein the eye gaze tracking system wirelessly interfaces with an external device.

## Claims

1. An eyelid position sensing system for a powered ophthalmic device, the eyelid position sensing system comprising:
a first electrode configured to be selectively overlaid by one or more of an upper eyelid and a lower eyelid of a user;
a second electrode configured to be selectively overlaid by one or more of an upper eyelid and a lower eyelid of a user; and
a system controller cooperatively associated with the first electrode and the second electrode to receive a capacitance measurement therefrom, the system controller configured to determine a position of one or more of the upper eyelid and the lower eyelid in spatial coordinates based on the capacitance measurement received from the first electrode and the second electrode.

2. The eyelid position sensing system according to claim 1, wherein the first electrode and the second electrode are spaced apart.

3. The eyelid position sensing system according to claim 1, wherein the first electrode and the second electrode are disposed on the powered ophthalmic device.

4. The eyelid position sensing system according to claim 3, wherein the powered ophthalmic device comprises a contact lens, an intraocular lens, an overlay lens, an ocular insert, or an optical insert, or a combination thereof.

5. The eyelid position sensing system according to any preceding claim, wherein one or more of the first electrode and the second electrode is configured in a generally curvilinear or annular shape.

6. The eyelid position sensing system according to claim 1, wherein the first electrode and the second electrode are disposed on opposite sides of a pupil of an eye of the user of the powered ophthalmic device.

7. The eyelid position sensing system according to any preceding claim, further comprising a signal processor configured to receive signals from the sensor, perform digital signal processing, and output one or more to the system controller.

8. The eyelid position sensing system according to claim 7, wherein signal processor comprises associated memory.

9. The eyelid position sensing system according to any preceding claim, further comprising a power supply.

10. The eyelid position sensing system according to any preceding claim, further comprising a transceiver configured to wirelessly interfaces with an external device.

11. A powered ophthalmic device comprising:
a lens including an optic zone and a peripheral zone; and
an eye gaze tracking system incorporated into the peripheral zone of the contact lens, the eye gaze tracking system including a capacitive touch sensor to detect a capacitance based at least on a position of one or more of an upper eyelid and a lower eyelid, a system controller cooperatively associated with the capacitive touch sensor, the system controller configured to determine gaze direction in spatial coordinates based on information received from the sensor, and at least one actuator configured to receive the output control signal and implement a predetermined function.

12. The powered ophthalmic device according to claim 11, wherein the capacitive touch sensor comprises a plurality of spaced-apart electrodes.

13. The powered ophthalmic device according to claim 12, wherein the plurality of spaced-apart electrodes are configured in a generally curvilinear or annular shape.

14. The powered ophthalmic device according to claim 12, wherein at least two of the plurality of spaced-apart electrodes are disposed on opposite sides of a pupil of an eye of the user of the powered ophthalmic device.

15. The powered ophthalmic lens according to any one of claims 11 to 14, wherein the eye gaze tracking system further comprises a signal processor configured to receive signals from the sensor, perform digital signal processing, and output one or more to the system controller.

16. The powered ophthalmic lens according to claim 15, wherein signal processor comprises associated memory.

17. The powered ophthalmic lens according to any one of claims 11 to 16, wherein the eye gaze tracking system further comprises a power supply.

18. The powered ophthalmic lens according to any one of claims 11 to 17, wherein the eye gaze tracking system further comprises a communication system for communication with at least a second lens.

19. The invention according to any preceding claim, wherein the spatial coordinates are in two dimensions.

20. The invention according to any one of claims 1 to 18, wherein the spatial coordinates are in three dimensions.

21. The powered ophthalmic lens according to any one of claims 11 to 18, wherein the eye gaze tracking system wirelessly interfaces with an external device.
